(19) Europäisches Patentamt
European Patent Office
Office européen des brevets

(11) Veröffentlichungsnummer: **0 228 598 B1**

# (12) EUROPÄISCHE PATENTSCHRIFT

(45) Veröffentlichungstag der Patentschrift: **06.11.91**

(51) Int. Cl.$^5$: **C07C 251/42**, C07C 251/52, C07C 251/54, A01N 35/10, A01N 35/06

(21) Anmeldenummer: **86116888.8**

(22) Anmeldetag: **04.12.86**

Die Akte enthält technische Angaben, die nach dem Eingang der Anmeldung eingereicht wurden und die nicht in dieser Patentschrift enthalten sind.

## (54) Cyclohexenonderivate, Verfahren zu ihrer Herstellung und ihre Verwendung als herbizide und das Pflanzenwachstum regulierende Mittel.

(30) Priorität: **09.12.85 DE 3543447**

(43) Veröffentlichungstag der Anmeldung:
**15.07.87 Patentblatt 87/29**

(45) Bekanntmachung des Hinweises auf die Patenterteilung:
**06.11.91 Patentblatt 91/45**

(84) Benannte Vertragsstaaten:
**AT BE CH DE ES FR GB GR IT LI NL**

(56) Entgegenhaltungen:
**EP-A- 0 088 299**
**EP-A- 0 123 001**
**EP-A- 0 124 041**
**EP-A- 0 126 713**

**PATENT ABSTRACTS OF JAPAN, Band 3, Nr. 87 (C-53), 25 Juli 1979, Seite 42 C 53**

(73) Patentinhaber: **BASF Aktiengesellschaft**
**Carl-Bosch-Strasse 38**
**W-6700 Ludwigshafen(DE)**

(72) Erfinder: **Meyer, Norbert, Dr.**
**Dossenheimer Weg 22**
**W-6802 Ladenburg(DE)**
Erfinder: **Jung, Johann, Prof.Dr.**
**Hardenburgstr. 19**
**W-6703 Limburgerhof(DE)**
Erfinder: **Rademacher, Wilhelm, Dr.**
**Austr. 1**
**W-6703 Limburgerhof(DE)**
Erfinder: **Kolassa, Dieter, Dr.**
**Moltkestr. 8**
**W-6700 Ludwigshafen(DE)**
Erfinder: **Becker, Rainer, Dr.**
**Im Haseneck 22**
**W-6702 Bad Duerkheim(DE)**
Erfinder: **Jahn, Dieter, Dr.**
**Burgunder Weg 8**
**W-6803 Edingen-Neckarhausen(DE)**

EP 0 228 598 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Keil, Michael, Dr.**
**Fontanestr. 4**
**W-6713 Freinsheim(DE)**
Erfinder: **Schirmer, Ulrich, Dr.**
**Berghalde 79**
**W-6900 Heidelberg(DE)**
Erfinder: **Wuerzer, Bruno, Dr.**
**Ruedigerstr. 13**
**W-6701 Otterstadt(DE)**

## Beschreibung

Die vorliegende Erfindung betrifft neue Cyclohexenonderivate, Verfahren zu ihrer Herstellung, herbizide und das Pflanzenwachstum regulierende Mittel, die die neuen Wirkstoffe enthalten sowie Verfahren zur Bekämpfung von unerwünschtem Pflanzenwachstum und zur Regulierung des Pflanzenwachstums.

Die herbizide Wirkung von Cyclohexenon-oximetherderivaten, die in 5-Stellung einen substituierten Cycloalkylrest aufweisen, ist bekannt DE-A-3 219 490 entspr. EP-A-88 299).

Weiterhin ist bekannt, daß bestimmte 2-Acyl-3-hydroxy-2-cyclohexen-1--one regulierend in das Pflanzenwachstum eingreifen (EP-A-123 001 und EP-A-126 713).

Es wurden nun neue Cyclohexenonderivate der Formel I

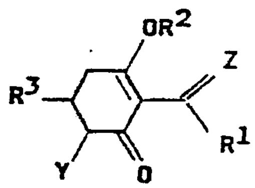

(I),

in der

- $R^1$ $C_1$-$C_4$-Alkyl,
- $R^2$ Wasserstoff, $C_1$-$C_{20}$-Alkylcarbonyl, $C_2$-$C_{20}$-Alkenylcarbonyl, insbesondere mit einer C-C-Doppelbindung, Benzoyl, das gegebenenfalls im Phenylring durch $C_1$-$C_8$-Alkyl substituiert ist, $C_1$-$C_4$-Trialkylsilyl, $C_1$-$C_7$-Alkylsulfonyl, Arylsulfonyl, $C_1$-$C_4$-Dialkylphosphono oder $C_1$-$C_4$-Dialkylthiophosphono,
- $R^3$ substituiertes Cyclohexyl, das bis zu 3 Methylgruppen tragen kann und das 1 oder 2 Substituenten enthält, ausgewählt aus der Gruppe, umfassend Hydroxy, $C_1$-$C_4$-Alkyl-carbonyloxy, $C_1$-$C_4$-Alkylcarbonylthio, Carboxymethylthio, $C_1$-$C_4$-Alkoxy-carbonylmethylthio, $C_1$-$C_4$-Alkylcarbamoyloxy, $C_1$-$C_4$-Dialkylcarbamoyloxy, $C_1$-$C_4$-Alkanoylamino, $C_3$-$C_6$-Cycloalkylcarbonylamino, $C_1$-$C_4$-Alkoxy, Aryloxy, $C_1$-$C_4$-Alkylthio, Arylthio, $C_3$-$C_4$-Alkoxyalkyl, $C_1$-$C_3$--Dialkylamino, $C_1$-$C_4$-Alkylsulfonyloxy, $C_1$-$C_4$-Alkylsulfinyloxy, $C_1$-$C_4$--Trialkylsilyl, $C_1$-$C_4$-Dialkylphosphono, Dimethoxymethyl, 1,3-Dioxolan--2-yl, 1,3-Dithiolan-2-yl, Halogen, Mercapto, $C_1$-$C_4$-Alkylthiocarbonyl, $C_1$-$C_4$-Alkoxycarbonyl, N-($C_1$-$C_4$-Alkyl)-carbamoyl, Carboxyl
- Y Wasserstoff, Methoxycarbonyl, Cyano, $C_1$-$C_4$-Alkyl oder Halogen und
- Z Sauerstoff oder den Rest $NOR^4$, in dem $R^4$ für $C_1$-$C_4$-Alkyl, $C_3$-$C_4$ Alkenyl, $C_3$-$C_4$-alkinyl, $C_2$-$C_4$-Halogenalkyl, $C_2$-$C_4$-Halogenalkenyl, insbesondere mit jeweils 1 bis 3 Halogenatomen, oder $C_2$-$C_4$-Alkoxyalkyl steht,

bedeuten, sowie Salze der Verbindungen, in denen $R^2$ Wasserstoff bedeutet, gefunden mit der Maßgabe, daß $R^3$ nicht für einen Cyclohexylrest steht, der durch einen $C_1$-$C_4$-Alkoxyrest oder ein Chloratom oder ein Bromatom substituiert ist.

Die neuen Cyclohexenonderivate der Formel I, in der Z für den Rest $NOR^4$ steht, besitzen eine gute herbizide Wirkung, vorzugsweise gegen Arten aus der Familie der Gräser (Gramineen).

Diejenigen Cyclohexenonderivate der Formel I, in der Z Sauerstoff bedeutet, weisen günstige wachstumsregulatorische Eigenschaften auf.

Die Verbindungen der Formel I können in mehreren tautomeren Formen auftreten, die alle vom Patentanspruch umfaßt werden. Beispielsweise können die Verbindungen mit $R^2$ = Wasserstoff und Z = $NOR^4$ u.a. in folgenden tautomeren Formen auftreten:

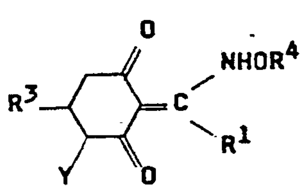

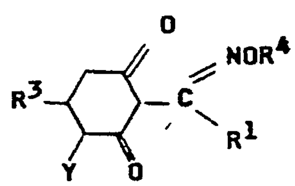

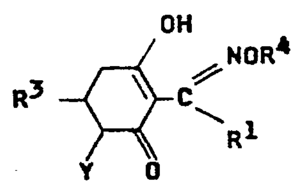

Wenn $R^4$ $C_2$-$C_4$-Halogenalkyl oder -alkenyl bedeutet, sollen diese Reste 1 bis 3 Halogenatome aufweisen. Weiterhin sind für den Fall, daß $R^4$ $C_3$-$C_4$-Alkenyl oder $C_2$-$C_4$-Halogenalkenyl bedeutet bei denjenigen Resten, bei denen E- und Z-Isomere auftreten können, beide Isomere vom Anspruch umfaßt.

$R^1$ in Formel I steht beispielsweise für Methyl, Ethyl, Propyl, Isopropyl, Butyl, sec-Butyl, Isobutyl oder tert-Butyl.

$R^2$ in Formel I steht beispielsweise für Wasserstoff, Acetyl, Propionyl, Acroyl, Butyryl, Isobutyryl, Pivaloyl, Valeroyl, 2-Ethylhexanoyl, Caproyl, Caprinoyl, Lauroyl, Palmitoyl, Stearoyl, Oleoyl, Benzoyl, 4-Methylbenzoyl, 4-Hexylbenzoyl, Trimethylsilyl, Triethylsilyl, Methylsulfonyl, Ethylsulfonyl, Benzoylsulfonyl, 4-Methylphenylsulfonyl, 2-Methylphenylsulfonyl, 2-Chlorphenylsulfonyl, 4-Chlorphenylsulfonyl, Diethylphosphono oder Diethylthiophosphono, insbesondere die vorstehend genannten $C_1$-$C_4$-Alkylcarbonylgruppen.
Grundkörper für die carbocyclischen Ringsysteme von $R^3$ in Formel I sind nicht-aromatische gegebenenfalls eine Doppelbindung enthaltende Ringe beispielsweise der Cyclopentan-, Cyclohexan-, Cycloheptan oder Cyclooctanring.

Beispielsweise bedeutet $R^3$ einen Cyclohexylring, der folgendes Substitutionsmuster aufweisen kann: 3,4-Dihydroxy, 3,4-Diacetoxy, 3,4-Dibutyryloxy, 3-Acetoxy-4-hydroxy-4-methyl, 3,4-Dihydroxy-4-methyl, 3-Acetylamino, 3,4-Dibenzoyloxy, 3-Benzoyloxy-4-hydroxy-4-methyl, 3,4-Bis(methylcarbamoyloxy), 3-Butyryloxy-4-hydroxy-4-methyl, 3-Benzoyloxy-4-hydroxy-4--methyl, 3-Dimethylamino-4-hydroxy-4-methylcyclohexyl, 4-Hydroxy-4-methyl--3-methylthio, 3-Acetylthio-4-hydroxy-4-methyl, 4-Hydroxy-4-methyl-3--methylthiocarbonyl, 3-Acetylthio-4-methyl, 4-Acetylthio, 4-Methylthiocarbonyl, 4-Mercapto, 3-Mercapto-4-methyl, 2-Methylthio, 3-Methylthio, 3-Hexylthio, 4-Methylthio, 4-Phenylthio, 3-Phenylthio, 4-Trimethylsilyl, 3-Trimethylsilyl, 4-Diethylphosphono, 4-Methylsulfonyloxy, 4-Acetoxy, 4-Benzoyloxy, 4-Hydroxy, 4-Carboxymethylthio, 4-Acetylamino, 4-Cyclopropylcarbonylamino, 4-Carboxyl, 4-Methoxycarbonyl, 4-Methylcarbamoyloxy, 4-(1,3-Dioxolan-2-yl), 4-Dimethoxymethyl, 4-Methoxymethoxy, 4-Methoxyethoxy, 3-Chlor, 3-Methoxymethyl-5-methyl, 5-Methoxymethyl-2-methyl oder 3-Ethylthio-4-hydroxy-4-methyl.

Für den Fall, daß in Formel I Z für die Gruppe $NOR^4$ steht, bedeutet $R^4$ beispielsweise Methyl, Ethyl, Propyl, Allyl, (E)-But-2-en-1-yl, Propargyl, 2-Chlorethyl, (E)-3-Chlorpropen-1-yl, Methoxymethyl oder 2-Methoxyethyl.

Als Salze der Verbindungen der Formel I, in der $R^2$ Wasserstoff bedeutet, kommen landwirtschaftlich brauchbare Salze, beispielsweise die Alkalimetallsalze, insbesondere die Kalium- oder Natriumsalze, Erdalkalimetallsalze, insbesondere Calcium- und Magnesiumsalze, Mangan-, Kupfer-, Zink-oder Eisensalze sowie Ammonium-, Sulfonium-, Sulfoxonium- und Phosphoniumsalze, beispielsweise Ammonium-, Tetraalkylammonium-, Benzyltrialkylammonium-, Trialkylsulfonium- oder Trialkylsulfoxoniumsalze in Betracht.

Bevorzugt sind Cyclohexenonderivate der Formel I, in denen $R^1$ $C_2$- oder $C_3$-Alkyl bedeutet.

Die Cyclohexenonderivate der Formel I, in der Z den Rest $NOR^4$ bedeutet, können durch Umsetzung derjenigen Cyclohexenonderivate der Formel I, in der Z für Sauerstoff steht, mit einer Ammoniumverbindung der Formel $R^4O$-$NH_3Y$, in der $R^4$ die obengenannte Bedeutung besitzt und Y ein Anion (z.B. Chlorid, Bromid oder Sulfat) bedeutet, erhalten werden.

Zweckmäßig führt man die Reaktion in heterogener Phase in einem inerten Verdünnungsmittel bei einer Temperatur von O bis 8O °C oder von O ° bis zum Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium oder Calcium. Außerdem können auch organische Basen, wie Pyridin oder tertiäre Amine, Verwendung finden. Die Base wird beispielsweise in Mengen von O,5 bis 2 Mol, bezogen auf die Ammoniumverbindung, zugesetzt.

Als Lösungsmittel sind beispielsweise Dimethylsulfoxid; Alkohole, wie Methanol, Ethanol oder Isopropanol; Benzol; gegebenenfalls chlorierte Kohlenwasserstoffe, wie Chloroform, Dichlorethan, Hexan oder Cyclohexan; Ester, wie Essigsäureethylester; oder Ether, wie Dioxan oder Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I (Z = $NOR^4$) können außerdem durch Umsetzen der Verbindungen der Formel I (Z = Sauerstoff) mit Hydroxylaminen der Formel $R^4O$-$NH_2$, in der $R^4$ die obengenannten Bedeutungen hat, in inerten Verdünnungsmitteln bei einer Temperatur von O °C bis 8O °C, insbesondere von 15 bis 7O °C, erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol oder Cyclohexanol; gegebenenfalls chlorierte Kohlenwasserstoffe, wie Hexan, Cyclohexan, Methylenchlorid, Toluol oder Dichlorethan; Ester, wie Essigsäureethylester; Nitrile, wie Acetonitril; cyclische Ether, wie Tetrahydrofuran.

Die Alkalimetallsalze der Verbindungen der Formel I (Z = $NOR^4$) können durch Behandeln dieser

Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen, Calcium-, Magnesium- und Bariumsalze können aus den Natriumsalzen durch Reaktion mit den entsprechenden Metallchloriden in wäßriger Lösung hergestellt werden. Die Ammonium-, Sulfonium-, Sulfoxonium- und Phosphoniumsalze lassen sich aus den Verbindungen der Formel I mittels Ammonium-, Sulfonium-, Sulfoxonium- oder Phosphoniumhydroxiden gegebenenfalls in wäßriger Lösung herstellen.

Diejenigen Cyclohexenonderivate der Formel I, in der Z für Sauerstoff steht, werden über die Zwischenstufe der Enolester erhalten. Dabei wird ein Cyclohexenonderivat der Formel II

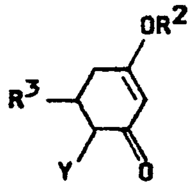

(II),

in der $R^2$, $R^3$ und Y die obengenannte Bedeutung besitzen mit einem Säurechlorid der Formel $R^1$-COCl, in der $R^1$ die obengenannte Bedeutung besitzt in Gegenwart einer Base (z.B. Triethylamin) in einem inerten Verdünnungsmittel (z.B. Tetrahydrofuran) umgesetzt und anschließend mit einem Imidazol- oder Pyridinderivat (z.B. 4-(N,N-Dimethylamino)pyridin) behandelt. Dieser Verfahrensschritt ist an sich bekannt und in der JP-A-63052/1979 beschrieben.

Es ist aber auch möglich, andere an sich bekannte Verfahrensmethoden, wie sie z.B. in Tetrahedron Letters 29, 2491 (1975) beschrieben sind, anzuwenden.

Zu den Verbindungen der Formel I gelangt man mittels an sich bekannter Verfahren, wie dies aus dem folgenden Schema hervorgeht:

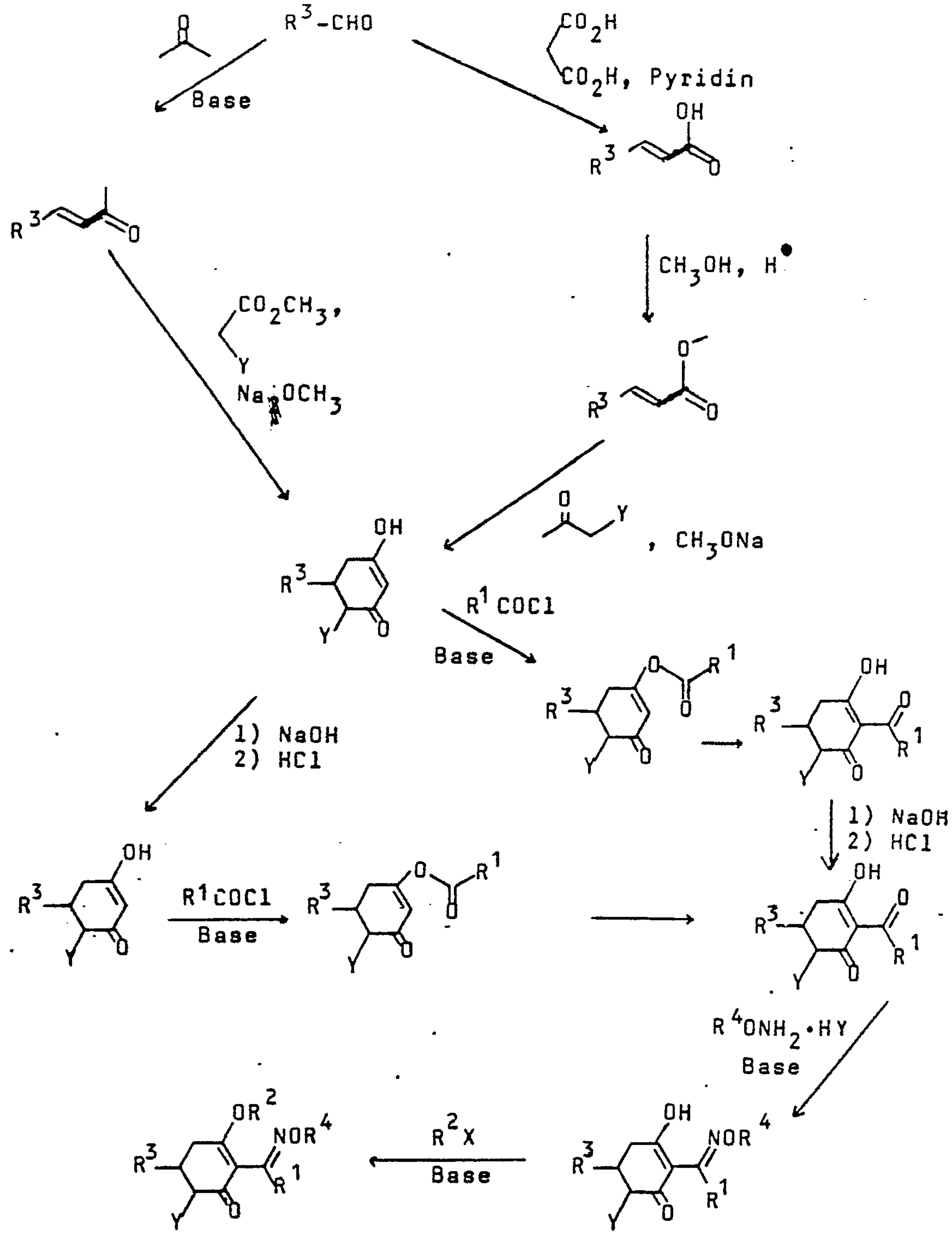


(Das im Schema aufgeführte offenkettige, alpha,beta-ungesättigte Keton sowie die ungesättigte Carbonsäure können sowohl als cis- wie auch als trans-Isomeres vorliegen.)

Zu Aldehyden der allgemeinen Formel $R^3$-CHO gelangt man nach literaturbekannten Verfahren, z.B. Oxidation von Alkoholen, Reduktion von Carbonsäurederivaten oder Hydroformylierung von Olefinen.

Die Einführung der funktionellen Gruppe(n) in dem Cyclohexylrest kann auf einer beliebigen Synthesestufe erfolgen, bevorzugt aber auf der Stufe der Verbindungen vom Typ der Formel I (mit A = Sauerstoff). Hierzu eignen sich alle literaturbekannten Methoden, z.B. Addition an eine Doppelbindung, nukleophile Ringöffnung von Oxiranen oder nukleophile Substitutionsreaktionen.

Die folgenden Beispiele sollen die Herstellung der Cyclohexenonderivate näher erläutern. In den Beispielen verhalten sich Gewichtsteile zu Volumenteilen wie Kilogramm zu Liter.

Beispiel A

30 Gewichtsteile 2-Butyryl-5-(3,4-epoxycyclohexyl)-3-hydroxy-2-cyclohexen-1-on und 130 Volumenteile 10 gew.%ige Natronlauge wurden bei Raumtemperatur gerührt, bis dünnschichtchromatographisch kein Ausgangsmaterial nachweisbar war. Die Lösung wurde mit konzentrierter Salzsäure angesäuert und mit Dichlormethan extrahiert. Die organische Phase wurde über Natriumsulfat getrocknet und das Lösungsmittel unter vermindertem Druck abdestilliert. Der Rückstand wurde säulenchromatographisch (Kieselgel, 230 - 400 mesh, Dichlormethan/Methanol = 200/1) gereinigt. Man erhielt 26 Gewichtsteile (81 %) 2-Butyryl-5-(3,4-dihydroxycyclohexyl)-3-hydroxy-2-cyclohexen-1-on (Verbindung Nr. 378) als gelbes Öl.

Beispiel B

4,4 Gewichtsteile 2-Butyryl-5-(3,4-dihydroxycyclohexyl)-3-hydroxy-2-cyclohexen-1-on, 1,6 Gewichtsteile Ethoxyamin, 1,4 Gewichtsteile Natriumhydrogencarbonat und 60 Volumenteile Methanol wurden bei Raumtemperatur 16 Stunden gerührt. Das Lösungsmittel wurde unter vermindertem Druck abdestilliert, zum verbleibenden Rückstand wurden jeweils 50 Volumenteile Wasser und Dichlormethan gegeben, nach heftigem Rühren die Phasen getrennt und die organische Phase über Natriumsulfat getrocknet. Nach Abdestillieren des Lösungsmittels unter vermindertem Druck erhielt man 4,5 Gewichtsteile (89 %) 5-(3,4-Dihydroxycyclohexyl)-2-[1-(ethoxyimino)-propyl]-3-hydroxy-2-cyclohexen-1-on (Verbindung Nr. 5).

In analoger Weise können die in Tabelle 1 aufgeführten Verbindungen der Formel I (Z = $NOR^4$) hergestellt werden.

Die $^1$H-NMR-Spektren wurden in Deuteriochloroform oder Hexadeuteriosulfoxid als Lösungsmittel mit Tetramethylsilan als innerem Standard aufgenommen. Die chemischen Verschiebungen werden in $\delta$ [ppm], registriert. Die Multiplizitäten werden wie folgt angegeben: s = Singulett, d = Dublett, t = Triplett, q = Quartett, m = Multiplett.

<u>Tabelle 1</u>

| Verbindung Nr. | $R^3$ | $R^2$ | $R^1$ | $R^4$ | Y | $^1$H-NMR-Daten |
|---|---|---|---|---|---|---|
| 1 | 3,4-Dihydroxycyclohexyl | H | Ethyl | Ethyl | H | 1.3(t,3H),2.6(m),4.1(q,2H) |
| 2 | 3,4-Dihydroxycyclohexyl | H | Ethyl | Allyl | H | 1.1(t,3H),2.9(q),4.55(d) |
| 3 | 3,4-Dihydroxycyclohexyl | H | Ethyl | (E)-2-Butenyl | H | 1.7(d),2.9(q),4.45(d) |
| 4 | 3,4-Dihydroxycyclohexyl | H | Ethyl | (E)-3-Chlor-2-propenyl | H | 2.9(q),3.55(m),3.7(m) |
| 5 | 3,4-Dihydroxycyclohexyl | H | Propyl | Ethyl | H | 1.3(t,3H),2.6(t,2H),4.05 (q,2H) |
| 6 | 3,4-Dihydroxycyclohexyl | H | Propyl | Allyl | H | 2.9(t,2H),3.6(m),3.7(m), 5.3(m) |
| 7 | 3,4-Dihydroxycyclohexyl | H | Propyl | (E)-2-Butenyl | H | 0.95(t),2.6(t),4.45(d) |
| 8 | 3,4-Dihydroxycyclohexyl | H | Propyl | (E)-3-Chlor-2-propenyl | H | 0.95(t,3H),2.85(t,2H), 4.55(d) |
| 9 | 3,4-Diacetoxycyclohexyl | H | Ethyl | Ethyl | H | 1.1(t),2.1(s),2.9(t) |
| 10 | 3,4-Diacetoxycyclohexyl | H | Ethyl | Allyl | H | |
| 11 | 3,4-Diacetoxycyclohexyl | H | Ethyl | (E)-2-Butenyl | H | |
| 12 | 3,4-Diacetoxycyclohexyl | H | Ethyl | (E)-3-Chlor-2-propenyl | H | 1.6(m),2.1(s),4.9(m) |
| 13 | 3,4-Diacetoxycyclohexyl | H | Propyl | Ethyl | H | 2.1(s),4.15(q),4.9(s), 5.0(s) |
| 14 | 3,4-Diacetoxycyclohexyl | H | Propyl | Allyl | H | 1.0(m),2.1(s),4.55(d) |
| 15 | 3,4-Diacetoxycyclohexyl | H | Propyl | (E)-2-Butenyl | H | 1.0(t),4.5(d),5.7(m) |
| 16 | 3,4-Diacetoxycyclohexyl | H | Propyl | (E)-3-Chlor-2-propenyl | H | 2.1(s),4.55(d),4.9(s), 5,0(s) |
| 17 | 3,4-Dibutyryloxycyclohexyl | H | Ethyl | Ethyl | H | |
| 18 | 3,4-Dibutyryloxycyclohexyl | H | Ethyl | Allyl | H | |
| 19 | 3,4-Dibutyryloxycyclohexyl | H | Ethyl | (E)-2-Butyl | H | |
| 20 | 3,4-Dibutyryloxycyclohexyl | H | Ethyl | (E)-3-Chlor-2-propenyl | H | 1.6(m),2.1(s),4.9(m) |
| 21 | 3,4-Dibutyryloxycyclohexyl | H | Propyl | Ethyl | H | 1.3(t),2.3(H),4.9(m),5.0(m) |
| 22 | 3,4-Dibutyryloxycyclohexyl | H | Propyl | Allyl | H | 1.7(m),2.3(t),5.35(m) |
| 23 | 3,4-Dibutyryloxycyclohexyl | H | Propyl | (E)-2-Butyl | H | |
| 24 | 3,4-Dibutyryloxycyclohexyl | H | Propyl | (E)-3-Chlor-2-propenyl | H | 1.0(t),2.9(m),4.5(d) |
| 25 | 3-Acetoxy-4-hydroxy-4--methylcyclohexyl | H | Ethyl | Ethyl | H | |
| 26 | 3-Acetoxy-4-hydroxy-4--methylcyclohexyl | H | Ethyl | Allyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 27 | 3-Acetoxy-4-hydroxy-4--methylcyclohexyl | H | Ethyl | (E)-2-Butyl | H | |
| 28 | 3-Acetoxy-4-hydroxy-4--methylcyclohexyl | H | Ethyl | (E)-3-Chlor-2-propenyl | H | |
| 29 | 3-Acetoxy-4-hydroxy-4--methylcyclohexyl | H | Propyl | Ethyl | H | 1.2(s),2.1(s),4.8(s) |
| 30 | 3-Acetoxy-4-hydroxy-4--methylcyclohexyl | H | Propyl | Allyl | H | |
| 31 | 3-Acetoxy-4-hydroxy-4--methylcyclohexyl | H | Propyl | (E)-2-Butenyl | H | |
| 32 | 3-Acetoxy-4-hydroxy-4--methylcyclohexyl | H | Propyl | (E)-3-Chlor-2-propenyl | H | 2.1(s),4.5(d),6.35(d) |
| 33 | 3,4-Dihydroxy-4-methyl-cyclohexyl | H | Ethyl | Ethyl | H | |
| 34 | 3,4-Dihydroxy-4-methyl--cyclohexyl | H | Ethyl | Allyl | H | |
| 35 | 3,4-Dihydroxy-4-methyl--cyclohexyl | H | Ethyl | (E)-2-Butenyl | H | |
| 36 | 3,4-Dihydroxy-4-methyl--cyclohexyl | H | Ethyl | (E)-3-Chlor-2-propenyl | H | |
| 37 | 3,4-Dihydroxy-4-methyl--cyclohexyl | H | Propyl | Ethyl | H | 1.3(m),3.6(m),4.1(q) |
| 38 | 3,4-Dihydroxy-4-methyl-cyclohexyl | H | Propyl | Allyl | H | |
| 39 | 3,4-Dihydroxy-4-methyl-cyclohexyl | H | Propyl | (E)-2-Butenyl | H | |
| 40 | 3,4-Dihydroxy-4-methyl-cyclohexyl | H | Propyl | (E)-3-Chlor-2-propenyl | H | |
| 41 | 3-Acetylaminocyclohexyl | H | Ethyl | Ethyl | H | |
| 42 | 3-Acetylaminocyclohexyl | H | Ethyl | Allyl | H | |
| 43 | 3-Acetylaminocyclohexyl | H | Ethyl | (E)-2-Butenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 44 | 3-Acetylaminocyclohexyl | H | Ethyl | (E)-3-Chlor-2-propenyl | H | |
| 45 | 3-Acetylaminocyclohexyl | H | Propyl | Ethyl | H | 0.95(t,3H),1.9(s,3H), 4.0(q,2H) |
| 46 | 3-Acetylaminocyclohexyl | H | Propyl | Allyl | H | |
| 47 | 3-Acetylaminocyclohexyl | H | Propyl | (E)-2-Butenyl | H | |
| 48 | 3-Acetylaminocyclohexyl | H | Propyl | (E)-3-Chlor-2-propenyl | H | |
| 49 | 3,4-Dibenzoyloxycyclohexyl | H | Ethyl | Ethyl | H | |
| 50 | 3,4-Dibenzoyloxycyclohexyl | H | Ethyl | Allyl | H | |
| 51 | 3,4-Dibenzoyloxycyclohexyl | H | Ethyl | (E)-2-Buten-1-yl | H | |
| 52 | 3,4-Dibenzoyloxycyclohexyl | H | Ethyl | (E)-3-Chlor-2-propenyl | H | |
| 53 | 3,4-Dibenzoyloxycyclohexyl | H | Propyl | Ethyl | H | |
| 54 | 3,4-Dibenzoyloxycyclohexyl | H | Propyl | Allyl | H | |
| 55 | 3,4-Dibenzoyloxycyclohexyl | H | Propyl | (E)-2-Butenyl | H | |
| 56 | 3,4-Dibenzoyloxycyclohexyl | H | Propyl | (E)-3-Chlor-2-propenyl | H | |
| 57 | 3-Benzoyloxy-4-hydroxy-4--methylcyclohexyl | H | Ethyl | Ethyl | H | |
| 58 | 3-Benzoyloxy-4-hydroxy-4--methylcyclohexyl | H | Ethyl | Allyl | H | |
| 59 | 3-Benzoyloxy-4-hydroxy-4--methylcyclohexyl | H | Ethyl | (E)-2-Butenyl | H | |
| 60 | 3-Benzoyloxy-4-hydroxy-4--methylcyclohexyl | H | Ethyl | (E)-3-Chlor-2-propenyl | H | |
| 61 | 3-Benzoyloxy-4-hydroxy-4--methylcyclohexyl | H | Propyl | Ethyl | H | 0.97(t), 1.30(s), 2.91(t) |
| 62 | 3-Benzoyloxy-4-hydroxy-4--methylcyclohexyl | H | Propyl | Allyl | H | |
| 63 | 3-Benzoyloxy-4-hydroxy-4--methylcyclohexyl | H | Propyl | (E)-2-Butenyl | H | |
| 64 | 3-Benzoyloxy-4-hydroxy-4--methylcyclohexyl | H | Propyl | (E)-3-Chlor-2-propenyl | H | |
| 65 | 3,4-Bis(methylcarbamoyloxy)-cyclohexyl | H | Ethyl | Ethyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 66 | 3,4-Bis(methylcarbamoyloxy)-cyclohexyl | H | Ethyl | Allyl | H | |
| 67 | 3,4-Bis(methylcarbamoyloxy)-cyclohexyl | H | Ethyl | (E)-2-Butenyl | H | |
| 68 | 3,4-Bis(methylcarbamoyloxy)-cyclohexyl | H | Ethyl | (E)-3-Chlor-2-propenyl | H | |
| 69 | 3,4-Bis(methylcarbamoyloxy)-cyclohexyl | H | Propyl | Ethyl | H | 1.3(t),2.8(d),4.1(q) |
| 70 | 3,4-Bis(methylcarbamoyloxy)-cyclohexyl | H | Propyl | Allyl | H | 2.8(d),4.5(d),5.4(m) |
| 71 | 3,4-Bis(methylcarbamoyloxy)-cyclohexyl | H | Propyl | (E)-2-Butenyl | H | 0.9(t),2.8(d),4.5(d) |
| 72 | 3,4-Bis(methylcarbamoyloxy)-cyclohexyl | H | Propyl | (E)-3-Chlor-2-propenyl | H | 0.95(t),2.8(d),6.3(d) |
| 73 | 3-Butyryloxy-4-hydroxy-4--methylcyclohexyl | H | Ethyl | Ethyl | H | |
| 74 | 3-Butyryloxy-4-hydroxy-4--methylcyclohexyl | H | Ethyl | Allyl | H | |
| 75 | 3-Butyryloxy-4-hydroxy-4--methylcyclohexyl | H | Ethyl | (E)-2-Butenyl | H | |
| 76 | 3-Butyryloxy-4-hydroxy-4--methylcyclohexyl | H | Ethyl | (E)-3-Chlor-2-propenyl | H | |
| 77 | 3-Butyryloxy-4-hydroxy-4--methylcyclohexyl | H | Propyl | Ethyl | H | |
| 78 | 3-Butyryloxy-4-hydroxy-4--methylcyclohexyl | H | Propyl | Allyl | H | |
| 79 | 3-Butyryloxy-4-hydroxy-4--methylcyclohexyl | H | Propyl | (E)-Butenyl | H | |
| 80 | 3-Butyryloxy-4-hydroxy-4--methylcyclohexyl | H | Propyl | (E)-3-Chlor-2-propenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 81 | 3-Benzoyloxy-4-hydroxy-4--methylcyclohexyl | H | Ethyl | Ethyl | H | |
| 82 | 3-Benzoyloxy-4-hydroxy-4--methylcyclohexyl | H | Ethyl | Allyl | H | |
| 83 | 3-Benzoyloxy-4-hydroxy-4--methylcyclohexyl | H | Ethyl | (E)-2-Butenyl | H | |
| 84 | 3-Benzoyloxy-4-hydroxy-4--methylcyclohexyl | H | Ethyl | (E)-3-Chlor-2-propenyl | H | |
| 85 | 3-Benzoyloxy-4-hydroxy-4--methylcyclohexyl | H | Propyl | Ethyl | H | |
| 86 | 3-Benzoyloxy-4-hydroxy-4--methylcyclohexyl | H | Propyl | Allyl | H | |
| 87 | 3-Benzoyloxy-4-hydroxy-4--methylcyclohexyl | H | Propyl | (E)-2-Butenyl | H | |
| 88 | 3-Benzoyloxy-4-hydroxy-4--methylcyclohexyl | H | Propyl | (E)-3-Chlor-2-propenyl | H | |
| 89 | 4-Hydroxy-4-methyl-3--methylthiocyclohexyl | H | Ethyl | Ethyl | H | |
| 90 | 4-Hydroxy-4-methyl-3-methyl--thiocyclohexyl | H | Ethyl | Allyl | H | |
| 91 | 4-Hydroxy-4-methyl-3-methyl--thiocyclohexyl | H | Ethyl | (E)-2-Butenyl | H | |
| 92 | 4-Hydroxy-4-methyl-3-methyl--thiocyclohexyl | H | Ethyl | (E)-3-Chlor-2-propenyl | H | |
| 93 | 4-Hydroxy-4-methyl-3-methyl--thiocyclohexyl | H | Propyl | Ethyl | H | |
| 94 | 4-Hydroxy-4-methyl-3--methylthiocyclohexyl | H | Propyl | Allyl | H | |
| 95 | 4-Hydroxy-4-methyl-3--methylthiocyclohexyl | H | Propyl | (E)-2-Butenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 96 | 4-Hydroxy-4-methyl-3-methylthiocyclohexyl | H | Propyl | (E)-3-Chlor-2-propenyl | H | |
| 97 | 3-Acetylthio-4-hydroxy-4-methylcyclohexyl | H | Ethyl | Ethyl | H | |
| 98 | 3-Acetylthio-4-hydroxy-4-methylcyclohexyl | H | Ethyl | Allyl | H | |
| 99 | 3-Acetylthio-4-hydroxy-4-methylcyclohexyl | H | Ethyl | (E)-2-Butenyl | H | |
| 100 | 3-Acetylthio-4-hydroxy-4-methylcyclohexyl | H | Ethyl | (E)-3-Chlor-2-propenyl | H | |
| 101 | 3-Acetylthio-4-hydroxy-4-methylcyclohexyl | H | Propyl | Ethyl | H | 1.28(s), 2.35(s), 4.09(g) |
| 102 | 3-Acetylthio-4-hydroxy-4-methylcyclohexyl | H | Propyl | Allyl | H | |
| 103 | 3-Acetylthio-4-hydroxy-4-methylcyclohexyl | H | Propyl | (E)-2-Butenyl | H | |
| 104 | 3-Acetylthio-4-hydroxy-4-methylcyclohexyl | H | Propyl | (E)-3-Chlor-2-propenyl | H | |
| 105 | 4-Hydroxy-4-methyl-3-methyl-thiocarbonylcyclohexyl | H | Ethyl | Ethyl | H | |
| 106 | 4-Hydroxy-4-methyl-3-methyl-thiocarbonylcyclohexyl | H | Ethyl | Allyl | H | |
| 107 | 4-Hydroxy-4-methyl-3-methyl-thiocarbonylcyclohexyl | H | Ethyl | (E)-2-Butenyl | H | |
| 108 | 4-Hydroxy-4-methyl-3-methyl-thiocarbonylcyclohexyl | H | Ethyl | (E)-3-Chlor-2-propenyl | H | |
| 109 | 4-Hydroxy-4-methyl-3-methyl-thiocarbonylcyclohexyl | H | Propyl | Ethyl | H | 0.96(t), 1.37(s), 4.10(g) |
| 110 | 4-Hydroxy-4-methyl-3-methyl-thiocarbonylcyclohexyl | H | Propyl | Allyl | H | 0.97(t), 1.38(s), 2.15(s) |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 111 | 4-Hydroxy-4-methyl-3-methylthiocarbonylcyclohexyl | H | Propyl | (E)-2-Butenyl | H | |
| 112 | 4-Hydroxy-4-methyl-3-methylthiocarbonylcyclohexyl | H | Propyl | (E)-3-Chlor-2-propenyl | H | 0.95(t), 2.15(s), 4.52(d) |
| 113 | 3-Acetylthio-4-methyl-cyclohexyl | H | Ethyl | Ethyl | H | |
| 114 | 3-Acetylthio-4-methyl-cyclohexyl | H | Ethyl | Allyl | H | |
| 115 | 3-Acetylthio-4-methylcyclohexyl | H | Ethyl | (E)-2-Butenyl | H | |
| 116 | 3-Acetylthio-4-methylcyclohexyl | H | Ethyl | (E)-3-Chlor-2-propenyl | H | |
| 117 | 3-Acetylthio-4-methylcyclohexyl | H | Propyl | Ethyl | H | 2.37(s), 3.98(s), 4.11(q) |
| 118 | 3-Acetylthio-4-methylcyclohexyl | H | Propyl | Allyl | H | |
| 119 | 3-Acetylthio-4-methylcyclohexyl | H | Propyl | (E)-2-Butenyl | H | |
| 120 | 3-Acetylthio-4-methylcyclohexyl | H | Propyl | (E)-3-Chlor-2-propenyl | H | |
| 121 | 3-Dimethylamino-4-hydroxy-4-methyl-cyclohexyl | H | Ethyl | Ethyl | H | |
| 122 | 3-Dimethylamino-4-hydroxy-4-methyl-cyclohexyl | H | Ethyl | Allyl | H | |
| 123 | 3-Dimethylamino-4-hydroxy-4-methyl-cyclohexyl | H | Ethyl | (E)-2-Butenyl | H | |
| 124 | 3-Dimethylamino-4-hydroxy-4-methyl-cyclohexyl | H | Ethyl | (E)-3-Chlor-2-propenyl | H | |
| 125 | 3-Dimethylamino-4-hydroxy-4-methyl-cyclohexyl | H | Propyl | Ethyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 126 | 3-Dimethylamino-4-hydroxy--4-methyl-cyclohexyl | H | Propyl | Allyl | H | |
| 127 | 3-Dimethylamino-4-hydroxy--4-methyl-cyclohexyl | H | Propyl | (E)-2-Butenyl | H | |
| 128 | 3-Dimethylamino-4-hydroxy--4-methyl-cyclohexyl | H | Propyl | (E)-3-Chlor-2-propenyl | H | |
| 129 | 4-Acetylthiocyclohexyl | H | Ethyl | Ethyl | H | |
| 130 | 4-Acetylthiocyclohexyl | H | Ethyl | Allyl | H | |
| 131 | 4-Acetylthiocyclohexyl | H | Ethyl | (E)-2-Butenyl | H | |
| 132 | 4-Acetylthiocyclohexyl | H | Ethyl | (E)-3-Chlor-2-propenyl | H | |
| 133 | 4-Acetylthiocyclohexyl | H | Propyl | Ethyl | H | 1.0(t),2.3(s),4.1(q) |
| 134 | 4-Acetylthiocyclohexyl | H | Propyl | Allyl | H | |
| 135 | 4-Acetylthiocyclohexyl | H | Propyl | (E)-2-Butenyl | H | |
| 136 | 4-Acetylthiocyclohexyl | H | Propyl | (E)-3-Chlor-2-propenyl | H | |
| 137 | 4-Methylthiocarbonylcyclohexyl | H | Ethyl | Ethyl | H | |
| 138 | 4-Methylthiocarbonylcyclohexyl | H | Ethyl | Allyl | H | |
| 139 | 4-Methylthiocarbonylcyclohexyl | H | Ethyl | (E)-2-Butenyl | H | |
| 140 | 4-Methylthiocarbonylcyclohexyl | H | Ethyl | (E)-3-Chlor-2-propenyl | H | |
| 141 | 4-Methylthiocarbonylcyclohexyl | H | Propyl | Ethyl | H | |
| 142 | 4-Methylthiocarbonylcyclohexyl | H | Propyl | Allyl | H | |
| 143 | 4-Methylthiocarbonylcyclohexyl | H | Propyl | (E)-2-Butenyl | H | |
| 144 | 4-Methylthiocarbonylcyclohexyl | H | Propyl | (E)-3-Chlor-2-propenyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 145 | 4-Mercaptocyclohexyl | H | Ethyl | Ethyl | H | |
| 146 | 4-Mercaptocyclohexyl | H | Ethyl | Allyl | H | |
| 147 | 4-Mercaptocyclohexyl | H | Ethyl | (E)-2-Butenyl | H | |
| 148 | 4-Mercaptocyclohexyl | H | Ethyl | (E)-3-Chlor-2-propenyl | H | |
| 149 | 4-Mercaptocyclohexyl | H | Propyl | Ethyl | H | |
| 150 | 4-Mercaptocyclohexyl | H | Propyl | Allyl | H | |
| 151 | 4-Mercaptocyclohexyl | H | Propyl | (E)-2-Butenyl | H | |
| 152 | 4-Mercaptocyclohexyl | H | Propyl | (E)-3-Chlor-2-propenyl | H | |
| 153 | 3-Mercapto-4-methylcyclohexyl | H | Ethyl | Ethyl | H | |
| 154 | 3-Mercapto-4-methylcyclohexyl | H | Ethyl | Allyl | H | 1.13(t), 2.90(g), 4.54(d) |
| 155 | 3-Mercapto-4-methylcyclohexyl | H | Ethyl | (E)-2-Butenyl | H | |
| 156 | 3-Mercapto-4-methylcyclohexyl | H | Ethyl | (E)-3-Chlor-2-propenyl | H | |
| 157 | 3-Mercapto-4-methylcyclohexyl | H | Propyl | Ethyl | H | |
| 158 | 3-Mercapto-4-methylcyclohexyl | H | Propyl | Allyl | H | |
| 159 | 3-Mercapto-4-methylcyclohexyl | H | Propyl | (E)-2-Butenyl | H | |
| 160 | 3-Mercapto-4-methylcyclohexyl | H | Propyl | (E)-3-Chlor-2-propenyl | H | |
| 161 | 3-Methylthiocyclohexyl | H | Ethyl | Ethyl | H | |
| 162 | 3-Methylthiocyclohexyl | H | Ethyl | Allyl | H | |
| 163 | 3-Methylthiocyclohexyl | H | Ethyl | (E)-2-Butenyl | H | |
| 164 | 3-Methylthiocyclohexyl | H | Ethyl | (E)-3-Chlor-2-propenyl | H | |
| 165 | 3-Methylthiocyclohexyl | H | Propyl | Ethyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 166 | 3-Methylthiocyclohexyl | H | Propyl | Allyl | H | |
| 167 | 3-Methylthiocyclohexyl | H | Propyl | (E)-2-Butenyl | H | |
| 168 | 3-Methylthiocyclohexyl | H | Propyl | (E)-3-Chlor-2-propenyl | H | |
| 169 | 4-Methylthiocyclohexyl | H | Ethyl | Ethyl | H | |
| 170 | 4-Methylthiocyclohexyl | H | Ethyl | Allyl | H | |
| 171 | 4-Methylthiocyclohexyl | H | Ethyl | (E)-2-Butenyl | H | |
| 172 | 4-Methylthiocyclohexyl | H | Ethyl | (E)-3-Chlor-2-propenyl | H | |
| 173 | 4-Methylthiocyclohexyl | H | Propyl | Ethyl | H | |
| 174 | 4-Methylthiocyclohexyl | H | Propyl | Allyl | H | |
| 175 | 4-Methylthiocyclohexyl | H | Propyl | (E)-2-Butenyl | H | |
| 176 | 4-Methylthiocyclohexyl | H | Propyl | (E)-3-Chlor-2-propenyl | H | |
| 177 | 4-Butylthiocyclohexyl | H | Ethyl | Ethyl | H | |
| 178 | 4-Butylthiocyclohexyl | H | Ethyl | Allyl | H | |
| 179 | 4-Butylthiocyclohexyl | H | Ethyl | (E)-2-Butenyl | H | |
| 180 | 4-Butylthiocyclohexyl | H | Ethyl | (E)-3-Chlor-2-propenyl | H | |
| 181 | 4-Butylthiocyclohexyl | H | Propyl | Ethyl | H | |
| 182 | 4-Butylthiocyclohexyl | H | Propyl | Allyl | H | |
| 183 | 4-Butylthiocyclohexyl | H | Propyl | (E)-2-Butenyl | H | |
| 184 | 4-Butylthiocyclohexyl | H | Propyl | (E)-3-Chlor-2-propenyl | H | |
| 185 | 4-Phenylthiocyclohexyl | H | Ethyl | Ethyl | H | |
| 186 | 4-Phenylthiocyclohexyl | H | Ethyl | Allyl | H | |
| 187 | 4-Phenylthiocyclohexyl | H | Ethyl | (E)-2-Butenyl | H | |
| 188 | 4-Phenylthiocyclohexyl | H | Ethyl | (E)-3-Chlor-2-propenyl | H | |
| 189 | 4-Phenylthiocyclohexyl | H | Propyl | Ethyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 190 | 4-Phenylthiocyclohexyl | H | Propyl | Allyl | H | |
| 191 | 4-Phenylthiocyclohexyl | H | Propyl | (E)-2-Butenyl | H | |
| 192 | 4-Phenylthiocyclohexyl | H | Propyl | (E)-3-Chlor-2-propenyl | H | |
| 193 | 3-Phenylthiocyclohexyl | H | Ethyl | Ethyl | H | |
| 194 | 3-Phenylthiocyclohexyl | H | Ethyl | Allyl | H | |
| 195 | 3-Phenylthiocyclohexyl | H | Ethyl | (E)-2-Butenyl | H | |
| 196 | 3-Phenylthiocyclohexyl | H | Ethyl | (E)-3-Chlor-2-propenyl | H | |
| 197 | 3-Phenylthiocyclohexyl | H | Propyl | Ethyl | H | |
| 198 | 3-Phenylthiocyclohexyl | H | Propyl | Allyl | H | |
| 199 | 3-Phenylthiocyclohexyl | H | Propyl | (E)-2-Butenyl | H | |
| 200 | 3-Phenylthiocyclohexyl | H | Propyl | (E)-3-Chlor-2-propenyl | H | |
| 201 | 4-Trimethylsilylcyclohexyl | H | Ethyl | Ethyl | H | |
| 202 | 4-Trimethylsilylcyclohexyl | H | Ethyl | Allyl | H | |
| 203 | 4-Trimethylsilylcyclohexyl | H | Ethyl | (E)-2-Butenyl | H | |
| 204 | 4-Trimethylsilylcyclohexyl | H | Ethyl | (E)-3-Chlor-2-propenyl | H | |
| 205 | 4-Trimethylsilylcyclohexyl | H | Propyl | Ethyl | H | |
| 206 | 4-Trimethylsilylcyclohexyl | H | Propyl | Allyl | H | |
| 207 | 4-Trimethylsilylcyclohexyl | H | Propyl | (E)-2-Butenyl | H | |
| 208 | 4-Trimethylsilylcyclohexyl | H | Propyl | (E)-3-Chlor-2-propenyl | H | |
| 209 | 3-Trimethylsilylcyclohexyl | H | Ethyl | Ethyl | H | |
| 210 | 3-Trimethylsilylcyclohexyl | H | Ethyl | Allyl | H | |
| 211 | 3-Trimethylsilylcyclohexyl | H | Ethyl | (E)-2-Butenyl | H | |
| 212 | 3-Trimethylsilylcyclohexyl | H | Ethyl | (E)-3-Chlor-2-propenyl | H | |
| 213 | 3-Trimethylsilylcyclohexyl | H | Propyl | Ethyl | H | |
| 214 | 3-Trimethylsilylcyclohexyl | H | Propyl | Allyl | H | |
| 215 | 3-Trimethylsilylcyclohexyl | H | Propyl | (E)-2-Butenyl | H | |
| 216 | 3-Trimethylsilylcyclohexyl | H | Propyl | (E)-3-Chlor-2-propenyl | H | |
| 217 | 4-Diethylphosphonocyclohexyl | H | Ethyl | Ethyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 218 | 4-Diethylphosphonocyclohexyl | H | Ethyl | Allyl | H | |
| 219 | 4-Diethylphosphonocyclohexyl | H | Ethyl | (E)-2-Butenyl | H | |
| 220 | 4-Diethylphosphonocyclohexyl | H | Ethyl | (E)-3-Chlor-2-propenyl | H | |
| 221 | 4-Diethylphosphonocyclohexyl | H | Propyl | Ethyl | H | |
| 222 | 4-Diethylphosphonocyclohexyl | H | Propyl | Allyl | H | |
| 223 | 4-Diethylphosphonocyclohexyl | H | Propyl | (E)-2-Butenyl | H | |
| 224 | 4-Diethylphosphonocyclohexyl | H | Propyl | (E)-3-Chlor-2-propenyl | H | |
| 225 | 4-Methylsulfonylcyclohexyl | H | Ethyl | Ethyl | H | |
| 226 | 4-Methylsulfonylcyclohexyl | H | Ethyl | Allyl | H | |
| 227 | 4-Methylsulfonylcyclohexyl | H | Ethyl | (E)-2-Butenyl | H | |
| 228 | 4-Methylsulfonylcyclohexyl | H | Ethyl | (E)-3-Chlor-2-propenyl | H | |
| 229 | 4-Methylsulfonylcyclohexyl | H | Propyl | Ethyl | H | |
| 230 | 4-Methylsulfonylcyclohexyl | H | Propyl | Allyl | H | |
| 231 | 4-Methylsulfonylcyclohexyl | H | Propyl | (E)-2-Butenyl | H | |
| 232 | 4-Methylsulfonylcyclohexyl | H | Propyl | (E)-3-Chlor-2-propenyl | H | |
| 233 | 4-Acetoxycyclohexyl | H | Ethyl | Ethyl | H | |
| 234 | 4-Acetoxycyclohexyl | H | Ethyl | Allyl | H | |
| 235 | 4-Acetoxycyclohexyl | H | Ethyl | (E)-2-Butenyl | H | |
| 236 | 4-Acetoxycyclohexyl | H | Ethyl | (E)-3-Chlor-2-propenyl | H | |
| 237 | 4-Acetoxycyclohexyl | H | Propyl | Ethyl | H | |
| 238 | 4-Acetoxycyclohexyl | H | Propyl | Allyl | H | |
| 239 | 4-Acetoxycyclohexyl | H | Propyl | (E)-2-Butenyl | H | |
| 240 | 4-Acetoxycyclohexyl | H | Propyl | (E)-3-Chlor-2-propenyl | H | |
| 241 | 4-Benzoyloxycyclohexyl | H | Ethyl | Ethyl | H | |
| 242 | 4-Benzoyloxycyclohexyl | H | Ethyl | Allyl | H | |
| 243 | 4-Benzoyloxycyclohexyl | H | Ethyl | (E)-2-Butenyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 244 | 4-Benzoyloxycyclohexyl | H | Ethyl | (E)-3-Chlor-2-propenyl | H | |
| 245 | 4-Benzoyloxycyclohexyl | H | Propyl | Ethyl | H | |
| 246 | 4-Benzoyloxycyclohexyl | H | Propyl | Allyl | H | |
| 247 | 4-Benzoyloxycyclohexyl | H | Propyl | (E)-2-Butenyl | H | |
| 248 | 4-Benzoyloxycyclohexyl | H | Propyl | (E)-3-Chlor-2-propenyl | H | |
| 249 | 4-Hydroxycyclohexyl | H | Ethyl | Ethyl | H | |
| 250 | 4-Hydroxycyclohexyl | H | Ethyl | Allyl | H | |
| 251 | 4-Hydroxycyclohexyl | H | Ethyl | (E)-2-Butenyl | H | |
| 252 | 4-Hydroxycyclohexyl | H | Ethyl | (E)-3-Chlor-2-propenyl | H | |
| 253 | 4-Hydroxycyclohexyl | H | Propyl | Ethyl | H | |
| 254 | 4-Hydroxycyclohexyl | H | Propyl | Allyl | H | |
| 255 | 4-Hydroxycyclohexyl | H | Propyl | (E)-2-Butenyl | H | |
| 256 | 4-Hydroxycyclohexyl | H | Propyl | (E)-3-Chlor-2-propenyl | H | |
| 257 | 4-Acetylaminocyclohexyl | H | Ethyl | Ethyl | H | |
| 258 | 4-Acetylaminocyclohexyl | H | Ethyl | Allyl | H | |
| 259 | 4-Acetylaminocyclohexyl | H | Ethyl | (E)-2-Butenyl | H | |
| 260 | 4-Acetylaminocyclohexyl | H | Ethyl | (E)-3-Chlor-2-propenyl | H | |
| 261 | 4-Acetylaminocyclohexyl | H | Propyl | Ethyl | H | |
| 262 | 4-Acetylaminocyclohexyl | H | Propyl | Allyl | H | |
| 263 | 4-Acetylaminocyclohexyl | H | Propyl | (E)-2-Butenyl | H | |
| 264 | 4-Acetylaminocyclohexyl | H | Propyl | (E)-3-Chlor-2-propenyl | H | |
| 265 | 4-Cyclopropylcarbonylamino-cyclohexyl | H | Ethyl | Ethyl | H | |
| 266 | 4-Cyclopropylcarbonylamino-cyclohexyl | H | Ethyl | Allyl | H | |
| 267 | 4-Cyclopropylcarbonylamino-cyclohexyl | H | Ethyl | (E)-2-Butenyl | H | |
| 268 | 4-Cyclopropylcarbonylamino-cyclohexyl | H | Ethyl | (E)-3-Chlor-2-propenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 269 | 4-Cyclopropylcarbonylamino-cyclohexyl | H | Propyl | Ethyl | H | |
| 270 | 4-Cyclopropylcarbonylamino-cyclohexyl | H | Propyl | Allyl | H | |
| 271 | 4-Cyclopropylcarbonylamino-cyclohexyl | H | Propyl | (E)-2-Butenyl | H | |
| 272 | 4-Cyclopropylcarbonylamino-cyclohexyl | H | Propyl | (E)-3-Chlor-2-propenyl | H | |
| 273 | 4-Carboxylcyclohexyl | H | Ethyl | Ethyl | H | |
| 274 | 4-Carboxylcyclohexyl | H | Ethyl | Allyl | H | |
| 275 | 4-Carboxylcyclohexyl | H | Ethyl | (E)-2-Butenyl | H | |
| 276 | 4-Carboxylcyclohexyl | H | Ethyl | (E)-3-Chlor-2-propenyl | H | |
| 277 | 4-Carboxylcyclohexyl | H | Propyl | Ethyl | H | |
| 278 | 4-Carboxylcyclohexyl | H | Propyl | Allyl | H | |
| 279 | 4-Carboxylcyclohexyl | H | Propyl | (E)-2-Butenyl | H | |
| 280 | 4-Carboxylcyclohexyl | H | Propyl | (E)-3-Chlor-2-propenyl | H | |
| 281 | 4-Methoxycarbonylcyclohexyl | H | Ethyl | Ethyl | H | |
| 282 | 4-Methoxycarbonylcyclohexyl | H | Ethyl | Allyl | H | |
| 283 | 4-Methoxycarbonylcyclohexyl | H | Ethyl | (E)-2-Butenyl | H | |
| 284 | 4-Methoxycarbonylcyclohexyl | H | Ethyl | (E)-3-Chlor-2-propenyl | H | |
| 285 | 4-Methoxycarbonylcyclohexyl | H | Propyl | Ethyl | H | |
| 286 | 4-Methoxycarbonylcyclohexyl | H | Propyl | Allyl | H | |
| 287 | 4-Methoxycarbonylcyclohexyl | H | Propyl | (E)-2-Butenyl | H | |
| 288 | 4-Methoxycarbonylcyclohexyl | H | Propyl | (E)-3-Chlor-2-propenyl | H | |
| 289 | 4-Methylcarbamoylcyclohexyl | H | Ethyl | Ethyl | H | |
| 290 | 4-Methylcarbamoylcyclohexyl | H | Ethyl | Allyl | H | |
| 291 | 4-Methylcarbamoylcyclohexyl | H | Ethyl | (E)-Butenyl | H | |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 292 | 4-Methylcarbamoylcyclohexyl | H | Ethyl | (E)-3-Chlor-2-propenyl | H | |
| 293 | 4-Methylcarbamoylcyclohexyl | H | Propyl | Ethyl | H | |
| 294 | 4-Methylcarbamoylcyclohexyl | H | Propyl | Allyl | H | |
| 295 | 4-Methylcarbamoylcyclohexyl | H | Propyl | (E)-2-Butenyl | H | |
| 296 | 4-Methylcarbamoylcyclohexyl | H | Propyl | (E)-3-Chlor-2-propenyl | H | |
| 297 | 4-(1,3-Dioxolan-2-yl)-cyclohexyl | H | Ethyl | Ethyl | H | |
| 298 | 4-(1,3-Dioxolan-2-yl)-cyclohexyl | H | Ethyl | Allyl | H | |
| 299 | 4-(1,3-Dioxolan-2-yl)-cyclohexyl | H | Ethyl | (E)-2-Butenyl | H | |
| 300 | 4-(1,3-Dioxolan-2-yl)-cyclohexyl | H | Ethyl | (E)-3-Chlor-2-propenyl | H | |
| 301 | 4-(1,3-Dioxolan-2-yl)-cyclohexyl | H | Propyl | Ethyl | H | |
| 302 | 4-(1,3-Dioxolan-2-yl)-cyclohexyl | H | Propyl | Allyl | H | |
| 303 | 4-(1,3-Dioxolan-2-yl)-cyclohexyl | H | Propyl | (E)-2-Butenyl | H | |
| 304 | 4-(1,3-Dioxolan-2-yl)-cyclohexyl | H | Propyl | (E)-3-Chlor-2-propenyl | H | |
| 305 | 4-Dimethoxymethylcyclohexyl | H | Ethyl | Ethyl | H | |
| 306 | 4-Dimethoxymethylcyclohexyl | H | Ethyl | Allyl | H | |
| 307 | 4-Dimethoxymethylcyclohexyl | H | Ethyl | (E)-2-Butenyl | H | |
| 308 | 4-Dimethoxymethylcyclohexyl | H | Ethyl | (E)-3-Chlor-2-propenyl | H | |
| 309 | 4-Dimethoxymethylcyclohexyl | H | Propyl | Ethyl | H | |
| 310 | 4-Dimethoxymethylcyclohexyl | H | Propyl | Allyl | H | |
| 311 | 4-Dimethoxymethylcyclohexyl | H | Propyl | (E)-2-Butenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 312 | 4-Dimethoxymethylcyclohexyl | H | Propyl | (E)-3-Chlor-2-propenyl | H | |
| 313 | 2-Methoxymethyl-5-methyl-cyclohexyl | H | Ethyl | Ethyl | H | |
| 314 | 2-Methoxymethyl-5-methyl-cyclohexyl | H | Ethyl | Allyl | H | |
| 315 | 2-Methoxymethyl-5-methyl-cyclohexyl | H | Ethyl | (E)-2-Butenyl | H | |
| 316 | 2-Methoxymethyl-5-methyl-cyclohexyl | H | Ethyl | (E)-3-Chlor-2-propenyl | H | |
| 317 | 2-Methoxymethyl-5-methyl-cyclohexyl | H | Propyl | Ethyl | H | |
| 318 | 2-Methoxymethyl-5-methyl-cyclohexyl | H | Propyl | Allyl | H | |
| 319 | 2-Methoxymethyl-5-methyl-cyclohexyl | H | Propyl | (E)-2-Butenyl | H | |
| 320 | 2-Methoxymethyl-5-methyl-cyclohexyl | H | Propyl | (E)-3-Chlor-2-propenyl | H | |
| 321 | 5-Methoxymethyl-2-methyl-cyclohexyl | H | Ethyl | Ethyl | H | |
| 322 | 5-Methoxymethyl-2-methyl-cyclohexyl | H | Ethyl | Allyl | H | |
| 323 | 5-Methoxymethyl-2-methyl-cyclohexyl | H | Ethyl | (E)-2-Butenyl | H | |
| 324 | 5-Methoxymethyl-2-methyl-cyclohexyl | H | Ethyl | (E)-3-Chlor-2-propenyl | H | |
| 325 | 5-Methoxymethyl-2-methyl-cyclohexyl | H | Propyl | Ethyl | H | |
| 326 | 5-Methoxymethyl-2-methyl-cyclohexyl | H | Propyl | Allyl | H | |
| 327 | 5-Methoxymethyl-2-methyl-cyclohexyl | H | Propyl | (E)-2-Butenyl | H | |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 328 | 5-Methoxymethyl-2-methyl-cyclohexyl | H | Propyl | (E)-3-Chlor-2-propenyl | H | |
| 329 | 3-Ethylthio-4-hydroxy-4-methylcyclohexyl | H | Ethyl | Ethyl | H | |
| 330 | 3-Ethylthio-4-hydroxy-4-methylcyclohexyl | H | Ethyl | Allyl | H | 1.34(s), 4.51(d), 6.0(m) |
| 331 | 3-Ethylthio-4-hydroxy-4-methylcyclohexyl | H | Ethyl | (E)-2-Butenyl | H | 1.15(t), 1.36(s), 4.45(d) |
| 332 | 3-Ethylthio-4-hydroxy-4-methylcyclohexyl | H | Ethyl | (E)-3-Chlor-2-propenyl | H | 1.35(s), 4.54(d), 6.37(d) |
| 333 | 3-Ethylthio-4-hydroxy-4-methylcyclohexyl | H | Propyl | Ethyl | H | 0.98(t), 4.12(q) |
| 334 | 3-Ethylthio-4-hydroxy-4-methylcyclohexyl | H | Propyl | Allyl | H | 0.97(t), 1.38(s), 4.56(d) |
| 335 | 3-Ethylthio-4-hydroxy-4-methylcyclohexyl | H | Propyl | (E)-2-Butenyl | H | |
| 336 | 3-Ethylthio-4-hydroxy-4-methylcyclohexyl | H | Propyl | (E)-3-Chlor-2-propenyl | H | 0.95(t), 1.34(s), 4.52(d) |

Tabelle 1 (Forts.)

| Verbindung Nr. | $R^3$ | $R^2$ | $R^1$ | $R^4$ | Y | $^1H$-NMR-Daten |
|---|---|---|---|---|---|---|
| 369 | 4-Carboxymethylthio-cyclohexyl | H | Ethyl | Ethyl | H | |
| 370 | 4-Carboxymethylthio-cyclohexyl | H | Ethyl | Allyl | H | |
| 371 | 4-Carboxymethylthio-cyclohexyl | H | Ethyl | (E)-2-Butenyl | H | |
| 372 | 4-Carboxymethylthio-cyclohexyl | H | Ethyl | (E)-3-Chlor-2-propenyl | H | |
| 373 | 4-Carboxymethylthio-cyclohexyl | H | Propyl | Ethyl | H | 2.9(t),3.3(s),4.1(q) |
| 374 | 4-Carboxymethylthio-cyclohexyl | H | Propyl | Allyl | H | |
| 375 | 4-Carboxymethylthio-cyclohexyl | H | Propyl | (E)-2-Butenyl | H | |
| 376 | 4-Carboxymethylthio-cyclohexyl | H | Propyl | (E)-3-Chlor-2-propenyl | H | |
| 377 | 3-Ethylthiocyclohexyl | H | Propyl | Ethyl | H | 1,0(t), 1,15-1,37 (2t), 4,1 (q) |
| 408 | 1-Methoxymethylcyclohex-1-yl | H | Propyl | Ethyl | H | 0.98(t), 3.28(s), 4.10(q) |

Tabelle 1 (Forts.)

| Verbindung Nr. | $R^3$ | $R^2$ | $R^1$ | $R^4$ | Y | $^1$H-NMR-Daten |
|---|---|---|---|---|---|---|
| 409 | 3,4-Bis(n-butoxycarbonyl)-cyclohexyl | H | Ethyl | (E)-3-Chlor-2-propenyl | H | 1.13(t), 4.53(d), 6.34(d) |
| 410 | 3,4-Bis(n-butoxycarbonyl)-cyclohexyl | H | Ethyl | Ethyl | H | 1.12(t), 2.91(m), 3.32(m) |
| 411 | 3,4-Bis(methoxycarbonyl)-cyclohexyl | H | Ethyl | Ethyl | H | 1.29(t), 3.67(s), 4.13(q) |
| 412 | 3,4-Bis(n-butoxycarbonyl)-cyclohexyl | H | Propyl | (E)-3-Chlor-2-propenyl | H | 0.93(m), 4.50(d), 6.33(d) |
| 413 | 3,4-Bis(n-butoxycarbonyl)-cyclohexyl | H | Propyl | Allyl | H | 3.86(m), 4.08(m), 4.53(d) |
| 414 | 3,4-Bis(n-butoxycarbonyl)-cyclohexyl | H | Propyl | Ethyl | H | 1.30(t), 3.86(m), 4.08(m) |
| 415 | 3-Butyryloxy-4-hydroxy-4-methyl-cyclohexyl | H | Ethyl | Allyl | H | 0.95(t), 1.98(s), 4.52(d) |
| 416 | 3-Butyryloxy-4-hydroxy-4-methyl-cyclohexyl | H | Ethyl | (E)-2-Butenyl) | H | 0.98(t), 1.19(s), 4.46(d) |
| 417 | " | H | Ethyl | (E)-3-Chlor-2-propenyl | H | 0.96(t), 1.18(s), 4.52(d) |

Analog Beispiel A können die in Tabelle 2 aufgeführten Verbindungen der Formel (Z = Sauerstoff) hergestellt werden.

Tabelle 2

| Verbindung Nr. | $R^3$ | $R^2$ | $R^1$ | Y | $^1$H-NMR-Daten |
|---|---|---|---|---|---|
| 378 | 3,4-Dihydroxycyclohexyl | H | Propyl | H | 0.97(t), 3.0(t), 3.5-3.75(m) |
| 379 | 3,4-Dihydroxycyclohexyl | H | Ethyl | H | |
| 380 | 3,4-Diacetoxycyclohexyl | H | Ethyl | H | |
| 381 | 3-Acetoxy-4-hydroxycyclohexyl | H | Propyl | H | |
| 382 | 3-Acetylaminocyclohexyl | H | Propyl | H | |
| 383 | 4-Acetylthiocyclohexyl | H | Propyl | H | |
| 384 | 4-Carboxymethylthio-cyclohexyl | H | Propyl | H | 0.97(t), 3.0(t), 3.34(s) |
| 385 | 3-Ethylthiocyclohexyl | H | Propyl | H | 0.97(t), 1.26(t), 3.02(t) |
| 386 | 1-Methoxymethylcyclohex-1-yl | H | Propyl | H | |
| 387 | 4-Acetylthiocyclohexyl | H | Propyl | H | 0.98(t), 2.32(s), 3.0(t) |
| 388 | 3-Acetylthio-4-methyl-cyclohexyl | H | Propyl | H | 2.36(s), 3.01(t), 3.98(s) |
| 389 | 3,4-Diacetoxycyclohexyl | H | Ethyl | H | 1.15(t), 2.06(s), 3.05(q) |
| 390 | 3,4-Bis(N-Methyl-carbamoyloxy)-cyclohexyl | H | Propyl | H | 0.98(t), 2.78(s), 2.97(t) |
| 391 | 3,4-Bis(n-butoxycarbonyl)-cyclohexyl | H | Ethyl | H | 1.14(t), 3.04(q), 4.10(m) |
| 392 | 3,4-Bis(methoxycarbonyl)-cyclohexyl | H | Ethyl | H | 1.17(t), 3.07(q), 3.73(s) |
| 393 | 3,4-Bis(n-butoxycarbonyl)-cyclohexyl | H | Propyl | H | 3.02(t), 3.87(m), 4.07(m) |

Tabelle 2 (Forts.)

| Verbindung Nr. | $R^3$ | $R^2$ | $R^1$ | Y | $^1$H-NMR-Daten |
|---|---|---|---|---|---|
| 394 | 3,4-Bis(methoxycarbonyl)-cyclohexyl | H | Propyl | H | 0.98(t), 3.33(m), 3.68(s) |
| 395 | 3,4-Bis(carboxyl)-cyclohexyl | H | Propyl | H | 0.93(t), 2.98(t), 3.24(m) |
| 396 | 3,4-Bis(carboxyl)-cyclohexyl | H | Ethyl | H | 1.04(t), 12.10(s), 17.95(s) |
| 397 | 3,4-Dihydroxy-4-methyl-cyclohexyl | H | Ethyl | H | 1.03(t), 2.98(q), 3.35(s) |
| 398 | 3,4-Dihydroxy-4-methyl-cyclohexyl | H | Propyl | H | 0.97(t), 3.00(t), 3.66(s) |
| 399 | 3-n-Butyryloxy-4-hydroxy-4-methyl-cyclohexyl | H | Ethyl | H | 1.17(s), 2.30(t), 3.05(q) |
| 400 | 3-Benzyoyloxy-4-hydroxy-4-methyl-cyclohexyl | H | Propyl | H | 1.23(s), 1.42(s), 5.09(s) |
| 401 | 4-Hydroxy-4-methyl-3-methylthio-cyclohexyl | H | Propyl | H | 0.98(t), 1.19(s), 3.01(t) |
| 402 | 3-Ethylthio-4-hydroxy-4-methyl-cyclohexyl | H | Ethyl | H | 1.13(t), 1.37(s), 3.04(q) |
| 403 | 3-Ethylthio-4-hydroxy-4-methyl-cyclohexyl | H | Propyl | H | 0.93(t), 1.18(s), 3.00(t) |
| 404 | 3-Acetylthio-4-hydroxy-4-methyl-cyclohexyl | H | Propyl | H | 0.98(t), 1.28(s), 3.79(s) |
| 405 | 3-Mercapto-4-methyl-cyclohexyl | H | Ethyl | H | 1.08(t), 1.13(s), 3.05(q) |
| 406 | 3-Acetylthio-4-methyl-cyclohexyl | H | Ethyl | H | 2.32(s), 3.05(q), 3.98(s) |
| 407 | 3,4-Bis(carboxy)-cyclohexyl | H | Propyl | H | 0.93(t), 2.98(t), 3.24(m) |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung

von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.% Wirkstoff.

Beispiele für Formulierungen sind:

I. Man vermischt 90 Gewichtsteile der Verbindung Nr. 5 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 37 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 15 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 7 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 12 werden mit 3 Gewichtsteilen des Natriumsalzes der Diisobutylnaphthalin-alpga-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 4 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 32 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 12 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gewichtsteile des Wirkstoffs Nr. 3 werden in 60 Gewichtsteilen einer Mischung, die aus 93 Gew.% Xylol und 7 Gew.% des Anlagerungsproduktes von 8 Mol Ethylenoxid an 1 Mol Nonylphenol besteht, gelöst. Man erhält eine Lösung, die 40 Gew.% des Wirkstoffs enthält.

Die Cyclohexenonderivate der Formel I (Z = $NOR^4$) besitzen eine gute herbizide Wirkung vorzugsweise gegen Arten aus der Familie der Gräser (Gramineen). Sie sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen wie Weizen und Reis selektiv verhalten und gleichzeitig unerwünschte Gräser bekämpfen.

Die Applikation der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen, und Wachstumsstadium 0,025 bis 3 kg/ha, vorzugsweise 0,05 bis 0,5 kg/ha.

Die Wirkung der Cyclohexenonderivate der Formel I (Z = $NOR^4$) auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern diese nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelte sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,03 bis 0,125 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Vergleichsperiode erstreckte sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

| Lat. Name | Deutscher Name |
|---|---|
| Alopecurus myosuroides | Ackerfuchsschwanz |
| Avena fatua | Flughafer |
| Avena sativa | Hafer |
| Digitaria sanguinalis | Blutfingerhirse |
| Echinochloa crus-galli | Hühnerhirse |
| Glycine max | Sojabohnen |
| Lolium multiflorum | Ital. Raygras |
| Medicago sativa | Luzerne |
| Setaria italica | Kolbenhirse |
| Sinapis alba | Weißer Senf |
| Sorghum halepense | Wilde Morenhirse |
| Zea mays | Mais |

Mit den beispielhaft ausgewählten Verbindungen Nr. 5, 7, 37, 16, 15, 12 und 9 lassen sich bei Vorauflaufapplikation von 3 kg Wirkstoff/ha Pflanzen aus der Familie der Gramineen gut bekämpfen. Senf als Beispielpflanze für breitblättrige Kulturpflanzen bleibt völlig unbeeinflußt.

Im Nachauflaufverfahren sind beispielsweise die Verbindungen Nr. 8 und 377 gut zur Bekämpfung unerwünschten Graswuchses geeignet. Die breitblättrige Kulturpflanze Luzerne zeigt dabei keinerlei Schädigung.

Weiterhin zeigen die beispielhaft ausgewählten Verbindungen Nr. 7, 4, 37 und 32 bei Nachauflaufbehandlung starke herbizide Aktivität gegen Beispielgräser, während die Soja als dikotyle Kulturpflanze völlig schadlos bleibt.

Schon mit niedrigen Aufwandmengen beispielsweise der Verbindungen Nr 12 und 3 läßt sich bei Nachauflaufbehandlung ein breites Gräserspektrum in Soja selektiv bekämpfen.

Beispielsweise zeigt die Verbindung Nr. 24 hohe herbizide Aktivität gegen unerwünschte Grasarten ohne die Kulturpflanze Weizen in ihrem Wachstum zu beeinflussen.

Unerwünschte grasartige Vegetation läßt sich beispielsweise mit den Verbindungen Nr. 71, 70 und 72 gut bekämpfen ohne die als Beispiel ausgewählte Kulturpflanze Luzerne zu schädigen.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |

| Botanischer Name | Deutscher Name |
|---|---|
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Cyclohexenonderivate der Formel I

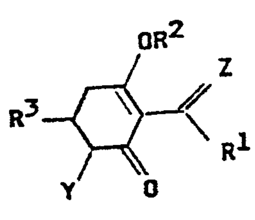 (I),

in der

$R^1$ $C_1$-$C_4$-Alkyl,

$R^2$ Wasserstoff, $C_1$-$C_{20}$-Alkylcarbonyl, $C_{2-20}$-Alkenylcarbonyl, Benzoyl, das gegebenenfalls im Phenylring durch $C_1$-$C_8$-Alkyl substituiert ist, $C_1$-$C_4$-Trialkylsilyl, $C_1$-$C_7$-Alkylsulfonyl, Arylsulfonyl, $C_1$-$C_4$-Dialkylphosphono oder $C_1$-$C_4$-Dialkylthio-phosphono.

$R^3$ substituiertes Cyclohexyl, das bis zu 3 Methylgruppen tragen kann und das 1 oder 2 Substituenten enthält, ausgewählt aus der Gruppe, umfassend
Hydroxy, $C_1$-$C_4$-Alkylcarbonyloxy, $C_1$-$C_4$-Alkylcarbonylthio, Carboxymethylthio, $C_1$-$C_4$-Alkoxycarbonylmethylthio, $C_1$-$C_4$-Alkylcarbamoyloxy, $C_1$-$C_4$-Dialkylcarbamoyloxy, $C_1$-$C_4$-Alkanoyla-

mino, $C_3$-$C_6$-Cycloalkylcarbonylamino, $C_1$-$C_4$-Alkoxy, Aryloxy, $C_1$-$C_4$-Alkylthio, Arylthio, $C_3$-$C_4$-Alkoxyalkyl, $C_1$-$C_3$-Dialkylamino, $C_1$-$C_4$-Alkylsulfonyloxy, $C_1$-$C_4$-Alkylsulfinyloxy, $C_1$-$C_4$-Trialkylsilyl, $C_1$-$C_4$-Dialkylphosphono, Dimethoxymethyl, 1,3-Dioxolan-2-yl, 1,3-Dithiolan-2-yl, Halogen, Mercapto, $C_1$-$C_4$-Alkylthiocarbonyl, $C_1$-$C_4$-Alkoxycarbonyl, N-($C_1$-$C_4$-Alkyl)-carbamoyl, Carboxyl

Y Wasserstoff, Methoxycarbonyl, Cyano, $C_1$-$C_4$-Alkyl oder Halogen und

Z Sauerstoff oder den Rest $NOR^4$, in dem $R^4$ für $C_1$-$C_4$-Alkyl, $C_3$-$C_4$-Alkenyl, $C_3$-$C_4$-Alkinyl, $C_2$-$C_4$-Halogenalkyl, $C_2$-$C_4$-Halogenalkenyl oder $C_2$-$C_4$-Alkoxyalkyl steht,

bedeuten sowie Salze der Verbindungen, in denen $R^2$ Wasserstoff bedeutet mit der Maßgabe, daß $R^3$ nicht für einen Cyclohexylrest steht, der durch einen $C_1$-$C_4$-Alkoxyrest oder ein Chloratom oder ein Bromatom substituiert ist.

2. Verfahren zur Herstellung von Cyclohexenonderivaten der Formel I gemäß Anspruch 1, in der Z Sauerstoff bedeutet, dadurch gekennzeichnet, daß man ein Cyclohexanderivat der Formel II

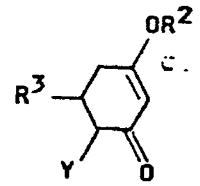 (II),

in der $R^2$, $R^3$ und Y die in Anspruch 1 genannte Bedeutung haben, zunächst mit einem Säurechlorid der Formel $R^1$-COCl, in der $R^1$ die in Anspruch 1 genannte Bedeutung besitzt, in Gegenwart einer Base umsetzt und anschließend mit einem Imidazol- oder Pyridinderivat behandelt.

3. Verfahren zur Herstellung von Cyclohexenonderivaten der Formel I gemäß Anspruch 1, in der Z den Rest $NOR^4$ bedeutet, dadurch gekennzeichnet, daß man ein Cyclohexenonderivat der Formel I gemäß Anspruch 1, in der Z Sauerstoff bedeutet

a) mit einer Ammoniumverbindung der Formel $R^4O$-$NH_3Y$, in der $R^4$ die in Anspruch 1 genannte Bedeutung hat und Y ein Anion bedeutet, in einem inerten Verdünnungsmittel in Gegenwart einer Base und gegebenenfalls in Gegenwart von Wasser bei einer Temperatur zwischen 0 und 80 °C oder

b) mit einem gegebenenfalls in wäßriger Lösung vorliegenden Hydroxylamin der Formel $R^4O$-$NH_2$, in der $R^4$ die in Anspruch 1 genannte Bedeutung hat, in einem inerten Lösungsmittel umsetzt.

4. Herbizid, enthaltend einen inerten Zusatzstoff und ein Cyclohexenonderivat der Formel I gemäß Anspruch 1, in der Z den Rest $NOR^4$ bedeutet.

5. Verfahren zur Bekämpfung von unerwünschtem Pflanzenwuchs, dadurch gekennzeichnet, daß man die unerwünschten Pflanzen und/oder die von unerwünschtem Pflanzenwachstum freizuhaltende Fläche mit einer herbizid wirksamen Menge eines Cyclohexenonderivats der Formel I gemäß Anspruch 1, in der Z den Rest $NOR^4$ bedeutet, behandelt.

6. Mittel zur Regulierung des Pflanzenwachstums, enthaltend einen inerten Zusatzstoff und ein Cyclohexenonderivat der Formel I gemäß Anspruch 1, in der Z Sauerstoff bedeutet.

7. Verfahren zur Regulierung des Pflanzenwachstums, dadurch gekennzeichnet, daß man ein Cyclohexenonderivat der Formel I gemäß Anspruch 1, in der Z Sauerstoff bedeutet, auf die Pflanzen und/oder deren Lebensraum einwirken läßt.

8. 5-(3,4-Dihydroxycyclohexyl)-2-[1-(ethoxyimino)-butyl]-3-hydroxy-2--cyclohexen-1-on.

9. 5-(3,4-Dihydroxycyclohexyl)-2-[1-(E-3-chlor-2-propenyloxyimino)--propyl]-3-hydroxy-2-cyclohexen-1-on.

10. Cyclohexenonderivate der Formel I gemäß Anspruch 1, in der $R^2$ $C_1$-$C_4$-Alkylcarbonyl bedeutet.

## Claims

1. A cyclohexenone derivative of the formula I

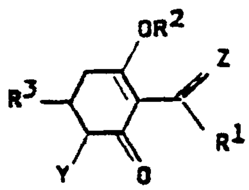

(I)

where

$R^1$ is $C_1$-$C_4$-alkyl,

$R^2$ is hydrogen, $C_1$-$C_{20}$-alkylcarbonyl, $C_2$-$C_{20}$-alkenylcarbonyl, or benzoyl which is unsubstituted or substituted in the phenyl ring by $C_1$-$C_8$-alkyl, or is $C_1$-$C_4$-trialkylsilyl, $C_1$-$C_7$-alkylsulfonyl, arylsulfonyl, $C_1$-$C_4$-dialkylphosphono or $C_1$-$C_4$-dialkylthiophosphono,

$R^3$ is substituted cyclohexyl which can carry up to 3 methyl groups and contains 1 or 2 substituents selected from the group consisting of hydroxyl, $C_1$-$C_4$-alkylcarbonyloxy, $C_1$-$C_4$-alkylcarbonylthio, carboxymethylthio, $C_1$-$C_4$-alkoxycarbonylmethylthio, $C_1$-$C_4$-alkylcarbamoyloxy, $C_1$-$C_4$-dialkylcarbamoyloxy, $C_1$-$C_4$-alkanoylamino, $C_3$-$C_6$-cycloalkylcarbonylamino, $C_1$-$C_4$-alkoxy, aryloxy, $C_1$-$C_4$-alkylthio, arylthio, $C_3$- or $C_4$-alkoxyalkyl, $C_1$-$C_3$-dialkylamino, $C_1$-$C_4$-alkylsulfonyloxy, $C_1$-$C_4$-alkylsulfinyloxy, $C_1$-$C_4$-trialkylsilyl, $C_1$-$C_4$-dialkylphosphono, dimethoxymethyl, 1,3-dioxolan-2-yl, 1,3-dithiolan-2-yl, halogen, mercapto, $C_1$-$C_4$-alkylthiocarbonyl, $C_1$-$C_4$-alkorycarbonyl, N-($C_1$-$C_4$-alkyl)-carbamoyl or carboxyl,

Y is hydrogen, methoxycarbonyl, cyano, $C_1$-$C_4$-alkyl or halogen and

Z is oxygen or a radical $NOR^4$, where $R^4$ is $C_1$-$C_4$-alkyl, $C_3$- or $C_4$-alkenyl, $C_3$- or $C_4$-alkynyl, $C_2$-$C_4$-haloalkyl, $C_2$-$C_4$-haloalkenyl or $C_2$-$C_4$-alkoxyalkyl,

or a salt of the compound in which $R^2$ is hydrogen, with the proviso that $R^3$ is not cyclohexyl which is substituted by $C_1$-$C_4$-alkoxy, chlorine or bromine.

2. A process for the preparation of a cyclohexenone derivative of the formula I as claimed in claim 1, wherein a cyclohexane derivative of the formula II

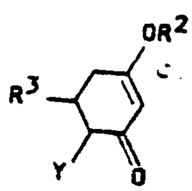

(II)

where $R^2$, $R^3$ and Y have the meanings stated in claim 1, is reacted initially with an acid chloride of the formula $R^1$-COCl, in which $R^1$ has the meanings stated in claim 1, in the presence of a base, and the product is then treated with an imidazole or pyridine derivative.

3. A process for the preparation of a cyclohexenone derivative of the formula I as claimed in claim 1, Z denoting $NOR^4$, wherein a cyclohexenone derivative of the formula I as claimed in claim 1, Z denoting oxygen, is reacted

a) with an ammonium compound of the formula $R^4O$-$NH_3Y$, $R^4$ having the meanings stated in claim 1 and Y denoting an anion, in an inert diluent, in the presence of a base, in the presence or absence of water, and at from 0° to 80° C, or

b) with a hydroxylamine - if desired, in aqueous solution - of the formula $R^4O$-$NH_2$, $R^4$ having the meanings stated in claim 1, in an inert solvent.

4. A herbicide containing an inert additive and a cyclohexenone derivative of the formula I as claimed in claim 1, Z denoting $NOR^4$.

5. A process for controlling undesirable plant growth, wherein the undesirable plants and/or the area to be kept free from undesirable plant growth are treated with a herbicidally effective amount of a cyclohexenone derivative of the formula I as claimed in claim 1, Z denoting $NOR^4$.

6. An agent for regulating plant growth, containing an inert additive and a cyclohexenone derivative of the formula I as claimed in claim 1, Z denoting oxygen.

7. A process for regulating plant growth, wherein a cyclohexenone derivative of the formula I as claimed in claim 1, Z denoting oxygen, is allowed to act on the plants and/or their habitat.

8. 5-(3,4-dihydroxycyclohexyl)-2-[1-(ethoxyimino)-butyl]-3-hydroxy-2-cyclohexen-1-one.

9. 5-(3,4-dihydroxycyclohexyl)-2-[1-(E-3-chloro-2-propenyl-oxyimino)-propyl]-3-hydroxy-2-cyclohexen-1-one.

10. A cyclohexenone derivative of the formula I as claimed in claim 1, where $R^2$ is $C_1$-$C_4$-alkylcarbonyl.

**Revendications**

1. Dérivés de cyclohexénone de formule I

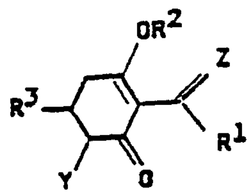

(I)

dans laquelle

$R^1$ représente un groupe alkyle en C 1-C 4,

R2 représente l'hydrogène, un groupe (alkyle en C 1-C 20)-carbonyle, (alcényle en C 2-C 20)-carbonyle, benzoyle éventuellement substitué dans le cycle phényle par des groupes alkyle en C 1-C 8, un groupe tri-(alkyle en C 1-C 4)-silyle, alkylsulfonyle en C 1-C 7, arylsulfonyle, di-(alkyle en C 1-C 4)-phosphono ou di-(alkyle en C 1-C 4)-thiophosphono,

$R^3$ représente un groupe cyclohexyle substitué qui peut porter jusqu'à trois groupes méthyle et porte un ou deux substituants choisis dans le groupé des suivants :

les groupes hydroxy, (alkyle en C 1-C 4)-carbonyloxy, (alkyle en C 1-C 4)-carbonylthio, carboxyméthylthio, (alcoxy en C 1-C 4)-carbonylméthylthio, (alkyle en C 1-C 4)-carbamoyloxy, di-(alkyle en C 1-C 4)-carbamoyloxy, alcanoylamino en C 1-C 4, (cycloalkyle en C 3-C 6)-carbonylamino, alcoxy en C 1-C 4, aryloxy, alkylthio en C 1-C 4, arylthio, alcoxyalkyle en C 3-C 4, di(alkyle en C 1-C 3)-amino, alkylsulfonyloxy en C 1-C 4, alkylsulfinyloxy en C 1-C 4, tri-(alkyle en C 1-C 4) silyle, di-(alkyle en C 1-C 4)-phosphono, diméthoxyméthyle, 1,3-dioxolanne-2-yle, 1,3-dithiolanne-2-yle, les halogènes, les groupes mercapto, (alkyle en C 1-C 4)-thiocarbonyle, (alcoxy en C 1-C 4)-carbonyle, N-(alkyle en C 1-C 4)-carbamoyle, carboxyle,

Y représente l'hydrogène, un groupe méthoxycarbonyle, cyano, alkyle en C 1-C 4 ou un halogène, et

Z représente l'oxygène ou le groupe $NOR^4$ dans lequel $R^4$ représente un groupe alkyle en C 1-C 4, alcényle en C 3-C4, alcynyle en C 3-C 4, halogénoalkyle en C 2-C 4, halogénoalcényle en C 2-C 4 ou alcoxyalkyle en C 2-C 4,

et les sels de ces composés pour lesquels $R^2$ représente l'hydrogène, sous réserve que $R^3$ ne peut représenter un groupe cyclohexyle substitué par un groupe alcoxy en C 1-C 4 ou par un atome de chlore ou de brome.

**2.** Procédé de préparation des dérivés de cyclohexénone de formule I de la revendication 1 dans laquelle Z représente l'oxygène, caractérisé en ce que l'on traite un dérivé du cyclohexane de formule II

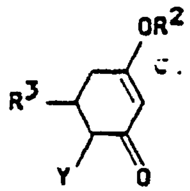

(II)

dans laquelle $R^2$, $R^3$ et Y ont les significations indiquées dans la revendication 1, d'abord par un chlorure d'acide de formule $R^1$-COC1 dans laquelle $R^1$ a les significations indiquées dans la revendication 1, en présence d'une base, puis par un dérivé d'imidazole ou de pyridine.

**3.** Procédé de préparation des dérivés de cyclohexénone de formule I de la revendication 1 dans laquelle Z représente le groupe $NOR^4$, caractérisé en ce que l'on fait réagir un dérivé de cyclohexénone de formule I de la revendication 1 dans laquelle Z représente l'oxygène,

a) avec un composé d'ammonium de formule $R^4O\text{-}NH_3Y$ dans laquelle $R^4$ a les significations indiquées dans la revendication 1 et Y représente un anion, dans un diluant inerte, en présence d'une base et le cas échéant en présence d'eau, à une température de 0 à 80 degrés C, ou bien
b) avec une hydroxylamine, éventuellement en solution aqueuse, de formule $R^4O\text{-}NH_2$ dans laquelle $R^4$ a les significations indiquées dans la revendication 1, dans un solvant inerte.

**4.** Produit herbicide contenant un additif inerte et un dérivé de cyclohexénone de formule I de la revendication 1 dans laquelle Z représente le groupe $NOR^4$.

**5.** Procédé pour combattre la croissance de végétaux indérisables, caractérisé en ce que l'on traite les végétaux indérisables et/ou l'aire à débarasser de la croissance des végétaux indésirables par une quantité herbicide efficace d'un dérivé de cyclohexénone de formule I selon la revendication 1, dans laquelle Z représente le groupe $NOR^4$.

**6.** Produit pour la régulation de la croissance des végétaux, contenant un additif inerte et un dérivé de cyclohexénone de formule I selon la revendication 1 dans laquelle Z représente l'oxygène.

**7.** Procédé pour la régulation de la croissance des végétaux, caractérisé en ce que l'on fait agir un dérivé de cyclohexénone de formule I de la revendication 1 dans laquelle Z représente l'oxygène sur les végétaux et/ou leur habitat.

**8.** La 5-(3,4-dihydroxycyclohexyl)-2- [1-(éthoxyimino)-butyl] -3hydroxy-2-cyclohexène-1-one.

**9.** La 5-(3,4-dihydroxycyclohexyl)-2- [1-(E-3-chloro-2-propényloxy-imino)-propyl] -3-hydroxy-2-cyclohexène-1-one.

**10.** Dérivés de cyclohexénones de formule I de la revendication 1 dans laquelle $R^2$ représente un groupe (alkyle en C 1-C 4)-carbonyle.